# EUROPEAN PATENT SPECIFICATION

(11) **EP 2 815 760 B1**
(45) Date of publication and mention of the grant of the patent: **09.10.2019**
(21) Application number: 13748698.1
(22) Date of filing: 15.02.2013
(51) Int. Cl.: A61K 31/198, A61P 9/00, A61P 9/10, A61P 15/10, A61P 21/00, A61P 21/02, A61K 38/06

(54) **AGENT FOR PREVENTING OR AMELIORATING VASCULAR ENDOTHELIAL MALFUNCTION**
MITTEL ZUR VERHINDERUNG ODER LINDERUNG EINER VASKULÄREN ENDOTHELIALEN FEHLFUNKTION
AGENT POUR PRÉVENIR OU AMÉLIORER UN DYSFONCTIONNEMENT DE L'ENDOTHÉLIUM VASCULAIRE

(30) Priority: 15.02.2012 JP 2012030849
(43) Date of publication of application: 24.12.2014
(73) Proprietor: Kyowa Hakko Bio Co., Ltd., Tokyo 100-8185 (JP)
(72) Inventor: MORITA, Masahiko, Tsukuba-shi Ibaraki 305-0841 (JP); KOMATSU, Miho, Tsukuba-shi Ibaraki 305-0841 (JP); HARA, Takahiro, Tsukuba-shi Ibaraki 305-0841 (JP)
(74) Representative: Vossius & Partner Patentanwälte Rechtsanwälte mbB
(86) International application number: PCT/JP2013/053661
(87) International publication number: WO 2013/122188

(56) References cited:
- WO-A1-2009/099132
- JP-A- 2001 507 696
- JP-A- 2004 521 083
- JP-A- 2006 117 645
- JP-A- 2006 273 789
- JP-A- 2006 273 789
- US-A1- 2001 056 068
- US-A1- 2001 056 068
- US-A1- 2009 047 340
- US-A1- 2009 047 340
- US-A1- 2011 044 965
- US-A1- 2011 044 965
- ABHIRAM PRASAD ET AL: "Endothelial Function Glutathione Reverses Endothelial Dysfunction and Improves Nitric Oxide Bioavailability", JOURNAL OF THE AMERICAN COLLEGE OF CARDIOLOGY, vol. 34, no. 2, 1 January 1999 (1999-01-01), pages 507-514, XP55159746,
- HAYASHI T JULIET P A R ET AL: "L-citrulline and L-arginine supplementation retards the proression of high-cholesterol-diet-induced atherosclerosis in rabbits", PROCEEDINGS OF THE NATIONAL ACADEMY OF SCIENCES, NATIONAL ACADEMY OF SCIENCES, US, vol. 102, no. 38, 20 September 2005 (2005-09-20), pages 13681-13686, XP008133300, ISSN: 0027-8424, DOI: 10.1073/PNAS.0506595102
- R SCHULZ: "Nitric oxide in myocardial ischemia/reperfusion injury", CARDIOVASCULAR RESEARCH, vol. 61, no. 3, 15 February 2004 (2004-02-15), pages 402-413, XP055212438, ISSN: 0008-6363, DOI: 10.1016/j.cardiores.2003.09.019
- TAGLIABUE ET AL: "Glutathione levels in patients with erectile dysfunction, with or without diabetes mellitus.", INT J ANDROL., vol. 28, no. 3, June 2005 (2005-06), pages 156-162, XP002744336,
- PRASAD,A. ET AL.: 'Glutathione reverses endothelial dysfunction and improves nitric oxide bioavailability' JOURNAL OF THE AMERICAN COLLEGE OF CARDIOLOGY vol. 34, no. 2, 1999, pages 507 - 14, XP055159746
- AKIO MATSUMOTO ET AL.: 'Igaku ni Okeru NO no Yakuwari' SHINDAN TO CHIRYO vol. 89, no. 1, 2001, pages 135 - 140, XP008174398
- HAYASHI,T. ET AL.: '1-Citrulline and 1-arginine supplementation retards the progression of high-cholesterol-diet-induced atherosclerosis in rabbits' PROCEEDINGS OF THE NATIONAL ACADEMY OF SCIENCES OF THE UNITED STATES OF AMERICA vol. 102, no. 38, 2005, pages 13681 - 6, XP008133300

## Description

### Technical Field

The present invention relates to an agent for preventing or ameliorating vascular endothelial malfunction having an improved effect of enhancing nitrogen monoxide (NO) production, which comprises citrulline or a salt thereof and glutathione or a salt thereof as active ingredients.

### Background Art

Citrulline is a type of amino acid existing in a free state, and is not used as material of protein synthesis in vivo. This compound plays important roles in the body, serving as an arginine precursor in arginine biosynthesis and a constituting factor of the NO cycle associated with NO supply.

NO produced by vascular endothelial cells exhibits a wide range of physiological activities for maintaining normal blood vessel functions, including vascular relaxation, oxidized LDL inhibition, platelet aggregation inhibition, smooth muscle cell antiproliferation, and anti-oxidation. Arteriosclerosis is a condition that involves loss of elasticity of the vascular wall due to increased inflammatory response in tunica intima and cholesterol accumulation. These conditions make it difficult to maintain a smooth blood flow and promote formation of blood clots. Many studies indicate lowered NO production in vascular endothelial cells as a cause of these conditions. Enhancing NO production in vascular endothelial cells is thus expected to prevent or ameliorate arteriosclerosis and other ischemic vascular diseases caused by vascular endothelial malfunction and promote blood flow.

There is a report that citrulline ingestion has an anti-arteriosclerosis effect and a blood flow ameliorating effect mediated by vasodilatation factor NO production (Non-Patent Document 1) and citrulline has been used primarily in the United States as a food material for NO production and blood flow improvement. In Europe, citrulline is used as an anti-fatigue drug in the form of citrulline malate.

Glutathione is a tripeptide consisting of glutamic acid, cysteine and glycine, and plays central roles in the mechanism of removing reactive oxygen species in vivo. This compound is also involved in the detoxication mechanism that removes foreign objects out of living body.

There are reports that glutathione ingestion has liver protective effect (Non-Patent Document 2) and whitening effect (Non-Patent Document 3). By taking advantage of these functions, glutathione has been used as a detoxicant for toxication, a drug for improving liver functions or a food material for anti-oxidation purposes.

However, it has not been known that glutathione has an effect for promoting NO production and that a synergistic effect for enhancing NO production can be obtained by combining glutathione and a salt thereof with citrulline or a salt thereof.

### Citation List

### Non-Patent Document

Non-Patent Document 1: PNAS, 2005, Vol. 102, p.13681-13686
Non-Patent Document 2: Journal of Nutritional Science & Vitaminology, 1998, Vol. 44, p. 613-624
Non-Patent Document 3: ARJINPATHANA et al., "Glutathione as an oral whitening agent: A randomized, double-blind, placebo-controlled study", Journal of Dermatological Treatment, 2012, 23: 97-102; Published online in 2010

### Summary of Invention

### Problems to be Solved by the Invention

Herein described is an agent for preventing or ameliorating vascular endothelial malfunction and arteriosclerosis-related symptoms caused by the progress of vascular endothelial malfunction (e.g., ischemic diseases such as myocardial infarction, angina, and peripheral artery occlusion) or low blood flow-related symptoms (e.g., stiff shoulders, excessive sensitivity to cold, swelling, erectile dysfunction, which has an improved NO production enhancing effect.

### Means for Solving the Problems

The present invention is defined as in the appended claims.

Also disclosed herein is
(1) An agent for enhancing NO production comprising citrulline or a salt thereof and glutathione or a salt thereof as active ingredients.
(2) An agent for preventing or ameliorating vascular endothelial malfunction, comprising citrulline or a salt thereof and glutathione or a salt thereof as active ingredients.
(3) An agent for preventing or ameliorating a symptom caused by vascular endothelial malfunction, comprising citrulline or a salt thereof and glutathione or a salt thereof as active ingredients.
(4) The agent for prevention or amelioration described in (3), wherein the symptom caused by vascular endothelial malfunction is at least one selected from an arteriosclerosis-related symptom and a low blood flow-related symptom.
(5) The agent for prevention or amelioration described in (4), wherein the arteriosclerosis-related symptom is ischemic disease.
(6) The agent for prevention or amelioration described in (5), wherein the ischemic disease is at least one selected from myocardial infarction, angina and peripheral artery occlusion.
(7) The agent for prevention or amelioration described in (4), wherein the low blood flow-related symptom is at least one selected from stiff shoulders, excessive sensitivity to cold, swelling, erectile dysfunction, rough skin and decrease in exercise performance due to skeletal muscle hypoactivity.

### Effects

Herein described is an agent for preventing or ameliorating vascular endothelial malfunction which is safe, effective, and has an improved enhancing effect on NO production, and comprises citrulline or a salt thereof and glutathione or a salt thereof as active ingredients.

### Brief Description of Drawings

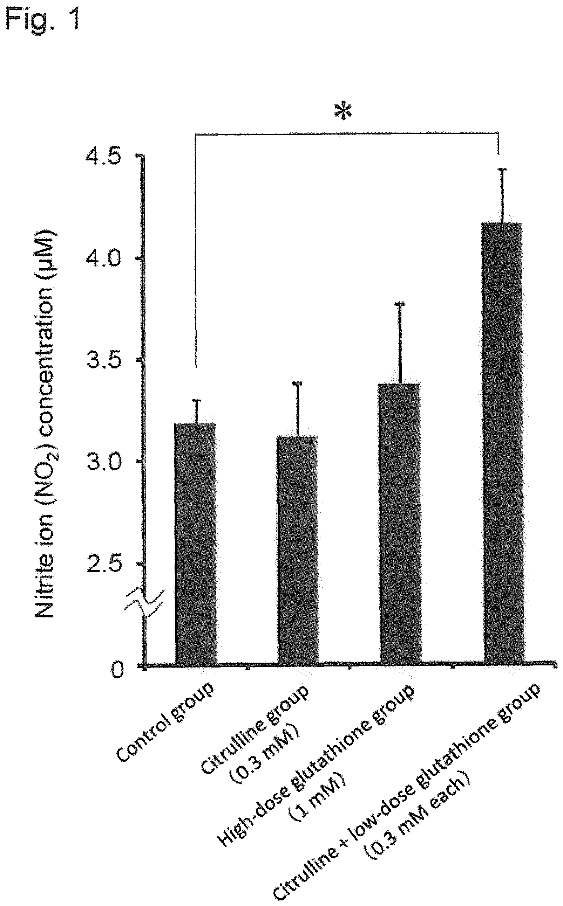

FIG. 1 is a diagram representing the concentration of nitrite (NO₂) which is the stable NO metabolite in media after adding different concentrations of samples to HUVEC. Mean ±SEM, N = 4, * indicates that the difference is significant for P < 0.05.

### Embodiments for Carrying Out the Invention

Citrulline used in the present invention includes L-citrulline or D-citrulline, and is preferably L-citrulline. Citrulline may be obtained by using various methods, including chemical synthesis, and fermentation. Citrulline may also be a commercially available product. Examples of chemical synthesis method used for citrulline production include the method described in J. Biol. Chem., 122, 477 (1938), J. Org. Chem., 6, 410 (1941). Examples of the fermentation method used for L-citrulline production include the methods described in JP-A-53-075387, and JP-A-63-068091. L-Citrulline and D-citrulline are also available from Sigma-Aldrich and other manufacturers.

Examples of citrulline salts include acid addition salts, metal salts, ammonium salts, organic amine addition salts, and amino acid addition salts. Examples of the acid addition salts include inorganic acid salts such as hydrochloride, sulfate, nitrate, and phosphate, and organic acid salts such as acetate, maleate, fumarate, citrate, malate, lactate, α-ketoglutarate, gluconate, and caprylate.

Examples of the metal salts include alkali metal salts, such as sodium salt and potassium salt; alkali-earth metal salts, such as magnesium salt and calcium salt; aluminum salts; and zinc salts.

Examples of the ammonium salts include ammonium salt and tetramethylammonium salt.

Examples of the organic amine addition salts include salts with morpholine and piperidine.

Examples of the amino acid addition salts include salts with glycine, phenylalanine, lysine, aspartic acid, and glutamic acid. Among the above salts of citrulline, malate is preferably used. It is, however, possible to use the other salts, or use two or more salts in appropriate combinations.

Glutathione used in the present invention may be reduced glutathione or oxidized glutathione.

The reduced glutathione refers to a tripeptide having the γ-L-Glu-L-Cys-Gly structure. The oxidized glutathione refers to a molecule with two reduced glutathione molecules attached to each other by S-S bonding.

The reduced glutathione and the oxidized glutathione used in the present invention may be one obtained by using any producing process. Examples of reduced glutathione producing processes include extraction from microorganisms such as yeast [Methods in Enzymology, 3, 603 (1957)], chemical synthesis [Bull.Chem. Soc. Jpn., 53, 2529 (1980)], and enzyme method (JP-A-61-74595). Examples of oxidized glutathione producing processes include the process described in Acta Biochim. Pol., 17, 175 (1970). Reduced glutathione and oxidized glutathione can also be purchased from Sigma-Aldrich and other manufacturers.

An agent for preventing or ameliorating vascular endothelial malfunction of the present invention may contain either one of the reduced glutathione and the oxidized glutathione alone, or may contain both the reduced glutathione and the oxidized glutathione at the same time.

The reduced glutathione and oxidized glutathione contained in the agent for preventing or ameliorating vascular endothelial malfunction of the present invention may be contained therein in the form of salts. Examples of the salts of reduced glutathione and oxidized glutathione include those described above in conjunction with the salts of citrulline.

Herein described is a substance which is metabolized into reduced glutathione in vivo, for example, N-acetylcysteine which can be used instead of glutathione.

The composition ratio of the citrulline or a salt thereof and the glutathione or a salt thereof contained in the agent for preventing or ameliorating vascular endothelial malfunction of the present invention is 1:100 to 100:1, preferably 1:50 to 50:1, particularly preferably 10:1 to 1:10 by weight.

The agent for preventing or ameliorating vascular endothelial malfunction of the present invention may be administered directly in the form of a citrulline or a salt thereof and a glutathione or a salt thereof. It is, however, typically more desirable to administer the agent for preventing or ameliorating vascular endothelial malfunction of the present invention in the form of various preparations.

The citrulline or a salt thereof, and the glutathione or a salt thereof are contained as active ingredients in such preparations, and preparations may further contain any other active ingredient. Such preparations are produced by mixing the active ingredients with one or more pharmaceutically acceptable carriers, using any method well known in the technical field of pharmaceuticals.

The administration route of the preparation is desirably one that is most effective for the prevention or amelioration of vascular endothelial malfunction. Examples include oral administration, and parenteral administration, such as intravenous, intraperitoneal, and subcutaneous administration. Preferred is oral administration.

The dosage form may be an oral form, such as a tablet, a powder, a granule, a pill, a suspension, an emulsion, an infusion/decoction, a capsule formulation, a syrup, a liquid, an elixir, an extract, a tincture, and a fluidextract, or a parenteral form such as an injection, a drop, a cream, and a suppository. Preferred is an oral form.

Liquid preparations, such as a syrup, suitable for oral administration may be prepared by adding water, sugars (e.g., sucrose, sorbitol, and fructose), glycols (e.g., polyethylene glycol, and propylene glycol), oils (e.g., sesame oil, olive oil, and soybean oil), antiseptics (e.g., p-hydroxybenzoic acid ester), paraoxybenzoic acid derivatives (e.g., methyl paraoxybenzoate), preservatives (e.g., sodium benzoate), flavors (e.g. strawberry flavor, and peppermint), and the like.

Tablets, powders, and granules suitable for oral administration may be prepared by adding excipient, such as sugars (e.g., lactose, white soft sugar, glucose, sucrose, mannitol, and sorbitol), starches (e.g., potato, wheat, and corn), inorganic materials (e.g., calcium carbonate, calcium sulfate, sodium hydrogencarbonate, and sodium chloride), crystalline cellulose, and plant powders (e.g. licorice powder and gentian powder), disintegrants, such as starch, agar, gelatin powder, crystalline cellulose, carmellose sodium, carmellose calcium, calcium carbonate, sodium bicarbonate, and sodium alginate, lubricants, such as magnesium stearate, talc, hydrogenated vegetable oil, Macrogol, and silicone oil, binders, such as polyvinyl alcohol, hydroxypropyl cellulose, methyl cellulose, ethyl cellulose, carmellose, gelatin, and starch paste, surfactants, such as fatty acid ester, and plasticizers, such as glycerine.

The preparations suitable for oral administration may contain additives generally used in foods and drinks, such as sweeteners, colors, preservatives, thickening agents, antioxidants, color forming agents, bleaching agents, anti-fungal agents, gum base, bittering agents, enzymes, brightening agents, acidulants, flavor enhancers, emulsifiers, toughening agents, production agents, flavors, and spice extracts.

The injections suitable for parenteral administration comprise a sterile aqueous agent that contains citrulline or a salt thereof, and glutathione or a salt thereof, and that is preferably isotonic to the recipient's blood. For example, in the case of injections, an injectable solution is prepared by using a carrier such as a salt solution, a glucose solution, and a mixture of a salt solution and a glucose solution.

As with the case of the oral form, the parenteral form also may contain one or more adjuvants selected from, for example, antiseptics, preservatives, excipients, disintegrants, lubricants, binders, surfactants, and plasticizers.

The concentrations of the citrulline or a salt thereof and the glutathione or a salt thereof in the agent for preventing or ameliorating vascular endothelial malfunction of the present invention are appropriately decided according to the type of preparation, the intended effect of the preparation administration, and the like. The concentration of citrulline or a salt thereof is typically 0.1 to 100 % by weight, preferably 0.5 to 80 % by weight, and particularly preferably 1 to 70 % by weight.

The dose and the dosing frequency of the agent for preventing or ameliorating vascular endothelial malfunction of the present invention depend on the dosage form, the age and the body weight of patients and the nature or severity of the symptoms in need of treatment. As a rule, the agent is given in a daily dose of typically 50 mg to 30 g, preferably 100 mg to 10 g, particularly preferably 200 mg to 3 g for adults in terms of a citrulline or a salt thereof, and a glutathione or a salt thereof, once to several times a day.

The administration period is not particularly limited, and is typically 1 day to 1 year, preferably 1 week to 3 months.

The agent for preventing or ameliorating vascular endothelial malfunction herein described may be used for the NO production-mediated prevention or amelioration of vascular endothelial malfunction. The agent for preventing or ameliorating vascular endothelial malfunction of the present invention can be used to prevent or ameliorate arteriosclerosis-related symptoms that occur with the progress of vascular endothelial malfunction and to prevent or ameliorate low blood flow-related symptoms. Examples of the effects that can be expected from the prevention or amelioration of arteriosclerosis-related symptoms that occur with the progress of vascular endothelial malfunction include prevention or amelioration of ischemic diseases such as myocardial infarction, angina and peripheral artery occlusion. Examples of the effects that can be expected from the prevention or amelioration of low blood flow-related symptoms include prevention or amelioration of stiff shoulders, excessive sensitivity to cold, swelling, erectile dysfunction, rough skin and decrease in exercise performance due to skeletal muscle hypoactivity.

Accordingly, individuals presenting with such symptoms can be relieved from such symptoms by administering the agent for preventing or ameliorating vascular endothelial malfunction.

Herein described are citrulline or a salt thereof, and glutathione or a salt thereof are used for the manufacture of the agent for preventing or ameliorating vascular endothelial malfunction.

Further, herein described is a method for elevating NO. The method of the present invention includes the step of administering citrulline or a salt thereof and glutathione or a salt thereof to a subject in need of NO elevation in amounts sufficient for elevating the NO level of the subject.

The following describes a test example concerning the effects of citrulline and glutathione on NO production.

### Test Example

A normal human umbilical vein endothelial cell (HUVEC) line available from Clonetics (SanDiego, CA, USA) was used with EGM-2 Bullet Kit medium containing 2% FBS (Takara Bio). The cells were cultured in at 37°C, in 5% CO₂ incubator, and used for experiment after 4 to 6 passages. Citrulline and reduced glutathione were obtained from Kyowa Hakko Bio.

A cell suspension (0.45 mL) with the adjusted initial cell concentration of 5 × 10⁵ cells/mL was inoculated in a 24-well plate (IWAKI). After 40-hour culture, the media were replaced with those containing citrulline (final concentration 0.3 mM: citrulline group), reduced glutathione (final concentration 1 mM: high-dose glutathione group), and a citrulline and reduced glutathione mixture (citrulline final concentration 0.3 mM; glutathione final concentration 0.3 mM: citrulline+low-dose glutathione group). Media with the sole addition of solvent (PBS) were used as a control group. After 24-hour culture, the cell culture (100 µL) was centrifuged at 12,000 rpm for 10 minutes, and the concentration of nitrite (NO₂), which is the stable NO metabolite, in culture supernatant was quantified by HPLC (ENO-20, EICOM).

MTT assay confirmed that the cell viability was unaffected in all of the addition groups tested.

Although it was known that citrulline would produce NO, the citrulline group and the high-dose glutathione group alone had no effect on NO production at the tested concentrations, as shown in FIG 1. However, the citrulline+low-dose glutathione group in which citrulline was used in combination with the glutathione added in a lower concentration than that used in the glutathione only group had a NO level about 31% higher than in the control group, showing a significant NO production promoting effect. These results show that the combined use of citrulline and glutathione synergically promotes NO production without affecting the survival rate and proliferative ability of vascular endothelial cells. The results also suggest that citrulline and glutathione, when combined, can desirably produce the agent for preventing or ameliorating vascular endothelial malfunction.

Examples of the present invention are described below.

### Example 1

### Production of Tablet Containing Citrulline and Glutathione

L-Citrulline (120 kg), reduced glutathione (120 kg), cyclic oligosaccharide (19 kg), cellulose (57 kg), and pullulan (1 kg) were granulated with a fluidized bed drying granulator. The resulting granulated material is mixed with calcium stearate (3 kg) by using a conical blender, and compression molded into tablets with a rotary compression molding machine.

### Example 2

### Production of Enteric Tablet Containing Citrulline and Glutathione

Surface of the tablets produced in Example 1 is coated with a shellac solution to produce enteric tablets.

### Example 3

### Production of Enteric Capsule Containing Citrulline and Glutathione

L-Citrulline (120 kg), reduced glutathione (120 kg), cyclic oligosaccharide (19 kg), cellulose (57 kg), calcium stearate (3 kg), and pullulan (1 kg) are mixed by using a conical blender. The resulting mixture (20 kg) is mixed and stirred with silicon dioxide (0.2 kg), and the resulting mixture is charged into a capsule filling machine to produce hard capsules filled with the mixture. Surface of the hard capsules is coated with a zein solution to produce enteric capsules.

### Example 4

### Production of Drink Containing Citrulline and Glutathione (1)

L-Citrulline (1.28 kg), oxidized glutathione (1.28 kg), erythritol (3 kg), citric acid (0.05 kg), artificial sweetener (3 g), and flavor (0.06 kg) are stirred and dissolved in water (50 L) at a liquid temperature of 70°C. After being adjusted to pH 3.3 with citric acid, the solution is sterilized with a plate sterilizer, and charged into a bottle. A drink is obtained after sterilization with a pasteuriser.

### Example 5

### Production of Drink Containing Citrulline and Glutathione (2)

Citrulline (20 mg), oxidized glutathione (20 mg), arginine (20 mg) are mixed with appropriate amounts of fructose glucose liquid sugar, common salt, citric acid, flavor, sodium citrate, calcium lactate, iron pyrophosphate, calcium gluconate, potassium chloride, magnesium chloride, and sweetener to produce a drink (555 ml).

### Example 6

### Production of Drink Containing Citrulline and Glutathione (3)

Citrulline (100 mg), oxidized glutathione (100 mg), arginine (100 mg), alanine (2.5 mg), glycine (2.5 mg), leucine (2.5 mg), isoleucine (1.3 mg), and valine (1.3 mg) are mixed with appropriate amounts of flavor and sweetener to produce a drink (300 ml).

### Example 7

### Production of Toner Containing Citrulline and Glutathione

Ethanol (10.0 % by weight), L-citrulline (2.0 % by weight), reduced glutathione (2.0 % by weight), 1,3-butylene glycol (5.0 % by weight), and purified water (83.0 % by weight) are mixed to produce a toner.

### Example 8

### Production of Cream Containing Citrulline and Glutathione

Polyethylene glycol (PGE55) monostearate (2.00 % by weight), self-emulsifying glyceryl monostearate (5.00 % by weight), cetyl alcohol (4.00 % by weight), squalane (6.00 % by weight), 2-ethylhexanoic acid triglyceride (6.00 % by weight), 1,3-butylene glycol (7.00 % by weight), L-histidine (3.00 % by weight), L-citrulline (1.00 % by weight), reduced glutathione (1.00 % by weight), and purified water (66.00 % by weight) are mixed to produce a cream.

### Example 9

### Production of Lotion Containing Citrulline and Glutathione

L-Citrulline (3.00 % by weight), oxidized glutathione (3.00 % by weight), L-serine (1.00 % by weight), water-soluble collagen (1% aqueous solution; 1.00 % by weight), sodium citrate (0.10 % by weight), citric acid (0.05 % by weight), licorice extract (0.20 % by weight), 1,3-butylene glycol (3.00 % by weight), and purified water (91.65 % by weight) are mixed to produce a lotion.

### Example 10

### Production of Mask Containing Citrulline and Glutathione

Polyvinyl alcohol (13.00 % by weight), L-aspartic acid (1.00 % by weight), L-citrulline (5.00 % by weight), reduced glutathione (5.00 % by weight), lauroyl hydroxyproline (1.00 % by weight), water-soluble collagen (1% aqueous solution; 2.00 % by weight), 1,3-butylene glycol (3.00 % by weight), ethanol (5.00 % by weight), and purified water (70.00 % by weight) are mixed to produce a mask.

### Example 11

### Production of Beauty Lotion Containing Citrulline and Glutathione

Hydroxyethyl cellulose (2% aqueous solution; 12.0 % by weight), xanthan gum (2% aqueous solution; 2.0 % by weight), L-citrulline (2.0 % by weight), reduced glutathione (2.0 % by weight), 1,3-butylene glycol (6.0 % by weight), concentrated glycerine (4.0 % by weight), sodium hyaluronate (1% aqueous solution; 5.0 % by weight), and purified water (69.0 % by weight) are mixed to produce a beauty lotion.

## Claims

1. An agent for use in preventing or ameliorating an ischemic disease, stiff shoulders due to vascular endothelial malfunction, excessive sensitivity to cold due to vascular endothelial malfunction, swelling due to vascular endothelial malfunction or erectile dysfunction, comprising citrulline or a salt thereof and glutathione or a salt thereof as active ingredients.

2. The agent for use according to claim 1, wherein the ischemic disease is at least one selected from myocardial infarction, angina and peripheral artery occlusion.

## Patentansprüche

1. Ein Mittel zur Verwendung bei der Vorbeugung oder Linderung einer ischämischen Erkrankung, steifen Schultern aufgrund einer vaskulären Endothelstörung, übermäßiger Empfindlichkeit gegenüber Kälte aufgrund einer vaskulären Endothelstörung, Schwellung aufgrund einer vaskulären Endothelstörung oder erektiler Dysfunktion, umfassend Citrullin oder ein Salz davon und Glutathion oder ein Salz davon als Wirkstoffe.

2. Das Mittel zur Verwendung nach Anspruch 1, wobei die ischämische Erkrankung mindestens eine ist, ausgewählt aus Myocardinfarkt, Angina und peripherem Arterienverschluss.

## Revendications

1. Agent pour utilisation dans la prévention ou l'amélioration d'une maladie ischémique, de raideurs d'épaule dues à un dysfonctionnement endothélial vasculaire, d'une sensibilité excessive au froid due à un dysfonctionnement endothélial vasculaire, d'un gonflement dû à un dysfonctionnement endothélial vasculaire ou d'un dysfonctionnement érectile, comprenant de la citrulline ou un sel de celle-ci et du glutathion ou un sel de celui-ci en tant qu'ingrédients actifs.

2. Agent pour utilisation selon la revendication 1, dans lequel la maladie ischémique est au moins une choisie parmi un infarctus du myocarde, angine et une occlusion artérielle périphérique.
